Europäisches Patentamt

European Patent Office

Office européen des brevets

(19)

(11) Veröffentlichungsnummer: **0 397 235**
**A2**

(12) **EUROPÄISCHE PATENTANMELDUNG**

(21) Anmeldenummer: **90201091.7**

(22) Anmeldetag: **23.04.90**

(51) Int. Cl.⁵: **G01N 33/38**

(30) Priorität: **05.05.89 DE 3914868**

(43) Veröffentlichungstag der Anmeldung:
**14.11.90 Patentblatt 90/46**

(84) Benannte Vertragsstaaten:
**AT FR GB IT**

(71) Anmelder: **Gerard, Helmut**
**Uhlandstrasse 2A**
**D-7898 Lauchringen(DE)**

(72) Erfinder: **Gerard, Helmut**
**Uhlandstrasse 2A**
**D-7898 Lauchringen(DE)**

(54) **Prüfgerät zur Bestimmung des Erstarrungszeitpunktes von Gips-Wasser-Gemischen.**

(57) Die bekannten Methoden werden manuell oder mit Apparaturen durchgeführt, welche wegen der Rheologie des Gipsbreies oder des großen apparativen Aufwandes unbefriedigende Ergebnisse oder Praktiken liefern. Die Erfindung erlaubt eine selbsttätige Bestimmung des Erstarrungszeitpunktes von Gips-Wasser- Gemische, und zwar unabhängig unterschiedlicher Konsistenzen und Rheologien.

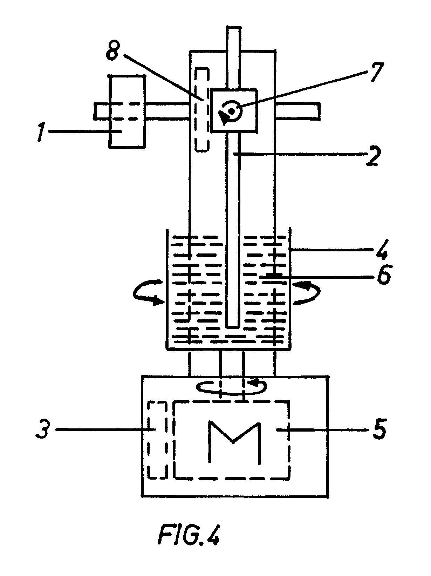

Bei dem Prüfgerät(Fig. 4) taucht ein schwenkbarer Eintauchstab(2) in ein rotierendes Gefäß(4) mit dem Gipsbrei(6). Durch ein Gegengewicht(1) wird eine Anpassung an die Konsistenz und Rheologie ermöglicht. Eintauchstab(2) und Gegengewicht(1) sind über die Drehwelle(7) verbunden. Bei Versteifungsbeginn schwenkt der Eintauchstab(2) aus. Mit dieser Schwenkung wird ein Endschalter(8) betätigt, welcher den Zeitnehmer(3) und den Motor(5) abschaltet.

Für die Verarbeitung von Gipsmörtel muß die Zeitdauer bis zum Versteifungsbeginn unabdingbar produktspezifisch eingestellt sein. Mit der Erfindung läßt sich werkseits ein Gipsmörtel für die Belange der Praxis auf die erforderliche Verarbeitungszeit genau einstellen.

FIG.4

## Prüfgerät zur Bestimmung des Erstarrungszeitpunktes von Gips-Wasser-Gemische.

Die Erfindung betrifft ein Prüfgerät für die Gips- und Gips-Trockenmörtel-Industrie zur selbsttätigen Bestimmung des Erstarrungszeitpunktes eines Gips-Wasser-Gemisches nach dem Oberbegriff des Anspruchs 1.

Nach DIN 1168 wird die beginnende Erhartung als Versteifungsbeginn bezeichnet. Bis zum Versteifungsbeginn ist die Zeit zu verstehen, innerhalb dieser das Gips-Wasser-Gemisch in seinen neuen, zukünftigen Formzustand gebracht werden kann. Die Zeit zwischen Versteifungsbeginn und -ende dient einer eventuellen Nachbearbeitung. Die Messung des Versteifungsbeginns wird nach DIN 1168 entweder mit der sogenannten Messerschnitt-Methode oder dem Vicatgerät unter Verwendung des Tauchkonus durchgeführt. Die Messungen erfolgen ausschließlich manuell.

Es fehlt nicht an Versuchen und wissenschaftlichen Arbeiten, die vorhandenen Ungenauigkeiten bei der Bestimmung des Hydratationsgrades oder -zustandes zu eliminieren und den Aussagewert zu verbessern, z. B.: Tonind.-Ztg. 97 (1973),Nr.1, S.15-17 und 21-24 Zement-Kalk-Gips-Nr. 5, 1975, S.233-239

Die Bestimmung des Erstarrungszeitpunktes soll so erfolgen, daß anhand des Erstarrungsgrades des Gips-Wasser-Gemisches ein Rückschluß auf die Zeitdauer der Verarbeitungsmöglichkeit zulässig ist. Wird gebrannter Gips mit Wasser gemischt, so hydratisiert dieser von seiner kristallwasserärmeren zur kristallwasserreicheren Form.

Das eingangs flüssige oder pastöse Gips-Wasser-Gemisch erfährt dadurch eine Ansteifung und Erhärtung.

Der hier erwähnten Erfindung liegt eine Meßmethode zugrunde welche ermöglicht, den Versteifungsbeginn eines Gips-Wasser-Gemisches - unabhängig von seiner Ausgangskonsistenz und Rheologie - genau zu bestimmen.

Die Meßaufgabe wird bei einer apparativgemäßen Einrichtung durch die kennzeichnenden Merkmale des Anspruches 1 gelöst. Vor Meßbeginn wird das Prüfgerät durch die Auswahl des Eintauchstabes, seiner Eintauchtiefe und Anbringung der Gegenkraft auf die Meßaufgabe vorbereitet. Entsprechend der Meßaufaufgabe wird zur Vereinfachung und Unverwechselbarkeit ein gekennzeichneter Stab und Gegenkraft verwendet.

Die mit der Erfindung erzielten Vorteile sind vielfältig: Durch Festlegung der Stabform, dessen Eintauchtiefe und Gegenkraft ist eine optimale Anpassung an die jeweils erforderliche Verarbeitungskonsistenz und Rheologie des zu prüfenden Gips-Wasser-Gemisches möglich.

Chemisch-modifizierte Trockengipsmörtel besitzen eine andere Rheologie, Konsistenz und Zähigkeit als die eines normalen Gips-Wasser-Gemisches. Mit diesem Prüfgerät kann der Versteifungsbeginn und somit die Verarbeitungszeit genau geprüft und den Erfordernissen der Praxis entsprechend mit dem Abbindeverzögerer eingestellt werden.

Das Prüfgerät arbeitet selbsttätig, ist unkompliziert und wartungsfrei in der Unterhaltung. Die Anschaffung ist kostengünstig.

Die Bedienung durch Hilfskräfte ist ebenso möglich wie die Anwendung außerhalb eines Labors am Ort der Verarbeitung des Gips-Wasser-Gemisches.

Für die Durchführung bisheriger Meßmethoden sind entweder komplizierte und teure Apparaturen notwendig, oder die Methoden erfassen das wichtige Kriterium der Bestimmung des praxisüblichen und erforderlichen Versteifungsbeginns zu ungenau oder garnicht.

Bei chemisch-modifizierten Gipsmörtel ergibt die Messerschnitt-Methode ungenaue und somit unbrauchbare Resultate. Die Vicat-Tauckonus-Methode ist zeitlich-manuell zu aufwendig.

Die Erfindung ist in den Zeichnungen (Seiten 6-7) dargestellt und wird im folgenden erläutert:

Fig. 1, Vorderansicht (Schnitt A-A)
Fig. 2, Seitenansicht (Schnitt B-B)
Fig. 3, Ansicht von oben

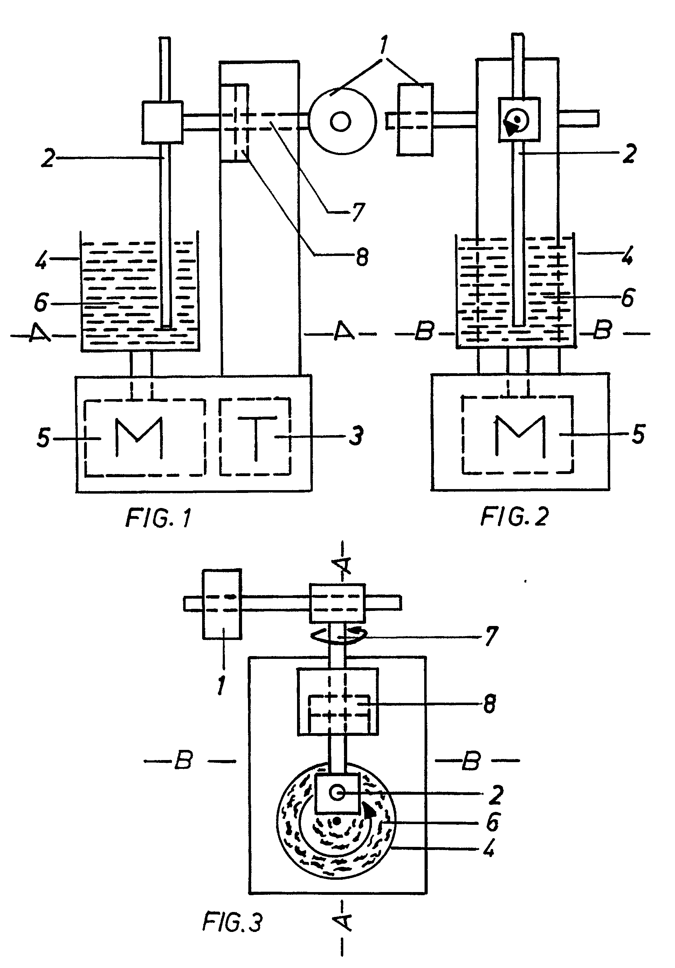

Das dem zu prüfenden Gipsmörtel zugeordnete Gegengewicht(1) und der Eintauchstab(2) werden am Prüfgerät eingestellt. Der Gips oder der Gipsmörtel wird mit der vorgeschriebenen Wassermenge angemischt und sofort der Zeitnehmer(3) am Prüfgerät gestartet. Der Gipsbrei wird in das Gefäß(4) eingefüllt, der Eintauchstab(2)auf die markierte Eintauchtiefe fixiert und der Motor(5) gestartet. Das Prüfgefäß(4) mit dem Gipsbrei(6) dreht am Eintauchstab(2) vorbei(Fig.3).

Bei Erreichung einer bestimmten Ansteifung des Gipsbreies erfahrt der Eintauchstab eine Auslenkung. Durch diese Drehbewegung wird der an der Welle(7) befestigte Endschalter(8) betätigt und schaltet den Motor(5) und Zeitnehmer(3) ab.

### Ansprüche

1. Prüfgerät zur Bestimmung des Erstarrungszeitpunktes von Gips- Wasser-Gemische dadurch gekennzeichnet, daß ein in das Gips-Wasser-Gemisch eintauchender Stab zum Erstarrungszeitpunkt des Gips-Wasser-Gemisches eine schwenkende oder drehende Bewegung erhält. dadurch gekennzeichnet, daß zur Erreichung der schwenkenden oder drehenden Bewegung des eintauchenden Stabes entweder das Gips-Wasser-Gemisch oder der Stab bewegt wird.

2. Prüfgerät nach 1,
dadurch gekennzeichnet, daß durch eine variable Gegenkraft zum schwenkenden oder drehenden Stab eine optimierte Anpassung an die Ausgangskonsistenz und Rheologie des Gips-Wasser-Gemisches möglich ist.

dadurch gekennzeichnet, das der schwenkende oder drehende Stab auf eine variable Eintauchtiefe in das Gips-Wasser-Gemisch eingestellt werden kann.

dadurch gekennzeichnet, daß der schwenkende oder drehende Stab sich außerhalb der Drehmittelachse des Gips-Wasser-Gemisches befindet.

dadurch gekennzeichnet, daß der schwenkende oder drehende Stab zugleich einen optisch oder akustisch wahrnehmbaren Zeitnehmer betätigt.

dadurch gekennzeichnet, daß der schwenkende oder drehende Stab zugleich den Stillstand des sich bewegenden Gips-Wasser-Gemisches oder sich seiner selbst durch einen mechanischen, elektromechanischen, optischen, elektronischen oder induktiven Schalter bewirkt.

dadurch gekennzeichnet, daß der schwenkende oder drehende Stab rund, flach, vieleckig, gerade oder gebogen sein kann.

FIG. 1

FIG. 2

FIG. 3

FIG.4